# EUROPEAN PATENT SPECIFICATION

(11) **EP 3 278 747 B1**
(45) Date of publication and mention of the grant of the patent: **10.07.2019**
(21) Application number: 17183700.8
(22) Date of filing: 28.07.2017
(51) Int. Cl.: A61B 17/29, A61B 17/00, A61B 90/00, A61B 18/14

(54) **LAPAROSCOPIC INSTRUMENT WITH RECESSED SEAL**
LAPAROSKOPISCHES INSTRUMENT MIT EINGELASSENER DICHTUNG
INSTRUMENT LAPAROSCOPIQUE AYANT UN JOINT EN RETRAIT

(30) Priority: 02.08.2016 US 201615226183
(43) Date of publication of application: 07.02.2018
(73) Proprietor: Microline Surgical, Inc., Beverly, MA 01915 (US)
(72) Inventor: JOSHI, Sharad H., Beverly, MA 01915 (US); MONIZ, Michael, Beverly, MA 01915 (US); BENTWOOD, Michael, Beverly, MA 01915 (US)
(74) Representative: Schaumburg und Partner Patentanwälte mbB

(56) References cited:
- EP-A1- 2 522 285
- US-A- 5 261 918
- US-A1- 2007 027 447

## Description

### BACKGROUND

### 1. Field of the Invention

This invention relates to an instrument including a surgical apparatus, such as a laparoscopic tube end used for performing laparoscopic, pelvoscopic, arthroscopic, thoroscopic and/or similar such procedures, and more particularly to an electrosurgical apparatus having a handpiece for fluidically sealing, isolating and electrically insulating a detachable tip when engaged with a laparoscopic tube end.

### 2. Background of the Invention

Medical procedures such as laparoscopy and the like, which employ a tip at the end of a tube for insertion into the patient, are beneficial because the incisions necessary to perform them are minimal in size, therefore promoting more rapid recovery and lower costs. For example, a patient who undergoes laparoscopic surgery may typically return to normal activity within a period of a few days to about a week, in contrast to more invasive procedures requiring a relatively larger incision (which may require about a month for recovery). Although the term "laparoscopic" is typically used hereinafter, such use of the term "laparoscopic" should be understood to encompass any such similar or related procedures such as, for example, arthroscopic, endoscopic, pelvoscopic and/or thoroscopic or the like, in which relatively small incisions are used.

However, when a tip is detachably connected to the tube end of a laparoscopic device, complications may occur if fluid breaches the connection and enters the interior of the tip or tube end. For example, septic contamination may arise in the laparoscopic device and/or electrical current may unintentionally leak therefrom. After each laparoscopic procedure, the device is also exposed to brushing, chemicals for cleaning and/or sterilization by various methods which may include heating, cooling, and flushing with additional chemicals. The seal in the handpiece may degrade to the point where it loses its integrity (either over use of hundreds of cycles, or from handpiece abuse; which can result in unintended cautery causing damage and injury to tissue around a surgical site).

Document EP 2522285 A1 discloses a connector for connecting an instrument tip with a laparoscopic tube end. The connector comprises a base forming a body of the connector, whereby a seal is permanently bonded to an inner surface of a proximal end of the base. The seal is configured to deform upon connection of the connector with the tube end.

Document US 5261918 A discloses a surgical grasping instrument assembled with a removable and disposable sheat member that prevents entrapment of tissue.

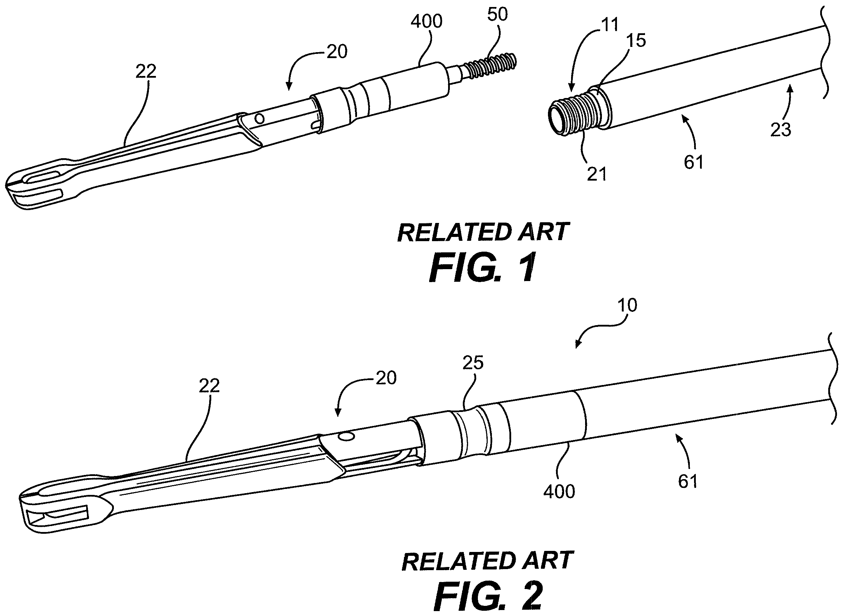

Figs. 1-2 shows a tube end assembly 11 of a reusable handpiece 23 to which a conventional tip (which may be a single-use device, or "SUD") 20 may be removably attached, thereby providing an instrument 10. The tip 20 has an end effector 22 in the form of a scissors, grasper and the like. The tube end assembly 11 distally extends from a tube 61, which in turn distally extends from the handpiece having handles (or other suitable controls, not shown, for actuating the tip 20) operable by the medical personnel or physician performing the surgery. The tube end assembly 11 includes an elastomeric tube end seal 15, which is typically made from EPDM (ethylene propylene diene monomer) and is in the form of an O-ring.

The seal 15 provides an electrically-safe seal between the interface of the tube end assembly 11 and the tip 20. The main purpose of the seal 15 is to prevent fluids from wicking or seeping into the seam between the two parts of the device (*i.e.,* the point of attachment of the tube end assembly 11 and the tip 20). The seal 15 is typically attached to the tube end assembly 11 via epoxy onto the tube end assembly so that the seal stays in place for multiple uses. One such seal is described in U.S. Patent Publication US 2007/0027447 A1. There are two potential problems with a seal of this configuration, namely: (1) it needs to be soft enough (*i.e.,* has a low durometer) to deform and create a seal, which means that
the seal 15 wears down quickly, resulting in the handpiece 23 being no longer usable; and (2) the epoxy seal between the seal 15 and the tube end assembly 11 eventually becomes brittle and cracks due to exposure to repeated sterilization cycles (*e.g.,* via chemical sterilization or autoclaving).

Once the integrity of the seal is lost, collateral damage can occur any time electrosurgical energy is applied to the device. In the illustrated device, the laparoscopic surgical tip 20 is assembled to the tube of the handpiece 23 using a dual-threaded threaded connector. In other words, the tip 20 includes a yoke 50 having external threading, which engages complementary internal threading on an actuation rod (not shown) extending along the length of the tube 61 (thereby providing for the actuation of the end effector), and a back hub 400 includes internal threading (not shown) which engages complimentary external threading 21 on the tube end assembly 11 to secure the back hub to the tube 61. The seal 15 on the handpiece 23 is compressed by the back hub to create a barrier for electricity and fluids.

### SUMMARY OF THE INVENTION

A feature of the disclosure provides handpiece for interfacing with an instrument tip. The handpiece may include a tube end having an interface at a distalmost end thereof and configured to attach to the instrument tip, the tube end having a retention seal channel about an exterior of the tube end, a retention seal fixedly positioned in the retention seal channel, the retention seal having a sealing ring channel about an exterior of the retention seal, an elastically-deformable sealing ring press-fit into the sealing ring channel such that the sealing ring is immovable in a longitudinal direction of the tube end, and an outer sheath positioned about the tube end such that the outer sheath and tube end sandwich the sealing ring and at least a portion of the retention seal.

The interface may be threaded and configured to attach to complimentary threads on the instrument tip. Also, the sealing ring may have a pentagonal cross section.

The retention seal may be made of a material which is softer than the tube end, and the sealing ring may be made of a material which is softer than the retention seal. The retention seal may further include a ridge positioned about a circumference of the tube end, the ridge further positioned, in the longitudinal direction of the tube end, between a distalmost end of the outer sheath and the interface. The ridge may be further configured to engage an inside of a back hub of the instrument tip and create a seal therewith.

The retention seal may further include a flange inwardly tapering toward a proximal end of the tube end, the flange positioned at a distalmost end of the outer sheath and configured to restrict movement of the outer sheath relative to the tube end. Also, the retention seal may be molded to the retention seal channel. Further, the retention seal may be made of plastic.

In the longitudinal direction of the tube end, the outside of the sealing ring may be downwardly tapered from a highest center point, and a lowest point of the outside of the sealing ring may be flush with the outside of the retention seal.

The tube end may further include a protrusion on the outside thereof, the retention seal may further include a recess, such that the protrusion engages with the recess.

Another feature of the disclosure provides an instrument having an instrument tip, and a handpiece for interfacing with the instrument tip. The handpiece may include a tube end having an interface at a distalmost end thereof and configured to removably attach to the instrument tip, the tube end having a retention seal channel about an exterior of the tube end, a retention seal fixedly positioned in the retention seal channel, the retention seal having a sealing ring channel about an exterior of the retention seal, an elastically-deformable sealing ring press-fit into the sealing ring channel such that the sealing ring may be immovable in a longitudinal direction of the tube end, and an outer sheath positioned about the tube end such that the outer sheath and tube end sandwich the sealing ring and at least a portion of the retention seal.

The instrument tip may include instrument tip threads, and the interface may include interface threads such that the interface threads threadedly engage with the instrument tip threads.

The instrument tip may further include a back hub, the retention seal may further include a ridge positioned about a circumference of the tube end, the ridge further positioned, in the longitudinal direction of the tube end, between a distalmost end of the outer sheath and the interface, and the ridge may be configured to engage an inside of the back hub and create a seal therewith.

The retention seal may further include a flange inwardly tapering toward a proximal end of the tube end, the flange positioned at a distalmost end of the outer sheath and configured to restrict movement of the outer sheath relative to the tube end. Also, the retention seal may be molded to the retention seal channel. Also, in the longitudinal direction of the tube end, the outside of the sealing ring may be downwardly tapered from a highest center point, and a lowest point of the outside of the sealing ring may be flush with the outside of the retention seal.

A further feature of the disclosure provides a method of assembling a handpiece for interfacing with an instrument tip. The method may include affixing a retention seal in a retention seal channel on an outside of a tube end, press-fitting an elastically-deformable sealing ring into a sealing ring channel on an outside of the retention seal, such that the sealing ring may be immovable in a longitudinal direction of the tube end, and affixing an outer sheath about the tube end such that the outer sheath and tube end sandwich the sealing ring and at least a portion of the retention seal. Also, the affixing of the retention seal comprises overmolding the retention seal to the outside of the tube end.

### BRIEF DESCRIPTION OF THE DRAWINGS

Fig. 1 shows a perspective view of an end of a reusable handpiece with a conventional seal;
Fig. 2 shows a perspective view of an assembled embodiment of a laparoscopic surgical tip of Fig. 1;
Fig. 3 show a partial cutaway view of a tube end assembly according to a feature of the disclosure;
Fig. 4 shows a side view of the tube end assembly according to a feature of the disclosure;
Fig. 5 shows a side sectional view of the tube end assembly according to a feature of the disclosure, taken along lines A-A of Fig. 4;
Fig. 6 shows a side sectional view of the tube end and retention seal according to a feature of the disclosure;
Fig. 7 shows a side view of the tube end and retention seal according to a feature of the disclosure; and
Fig. 8 shows a perspective view of the sealing ring according to a feature of the disclosure.

### DETAILED DESCRIPTION

The particulars shown herein are by way of example and for purposes of illustrative discussion of the embodiments of the present invention only, and are presented for providing what is believed to be the most useful and readily understood description of the principles and conceptual aspects of the present invention. In this regard, no attempt is made to show structural details of the present invention in more detail than is necessary for the fundamental understanding of the present invention, the description taken with the drawings making apparent to those skilled in the art how the several forms of the present invention may be embodied in practice.

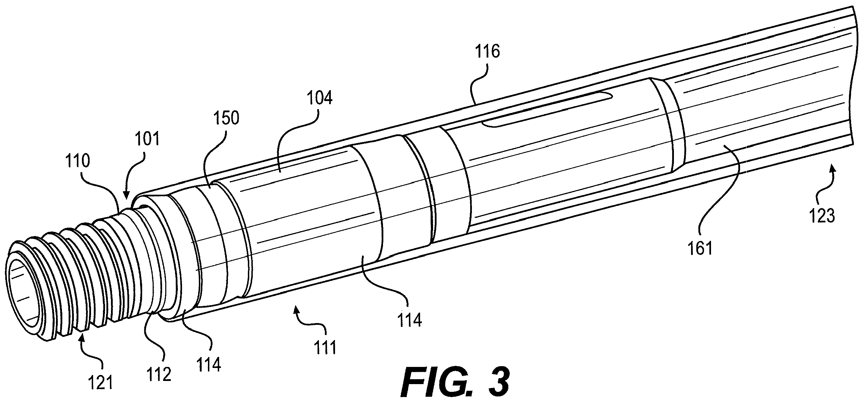

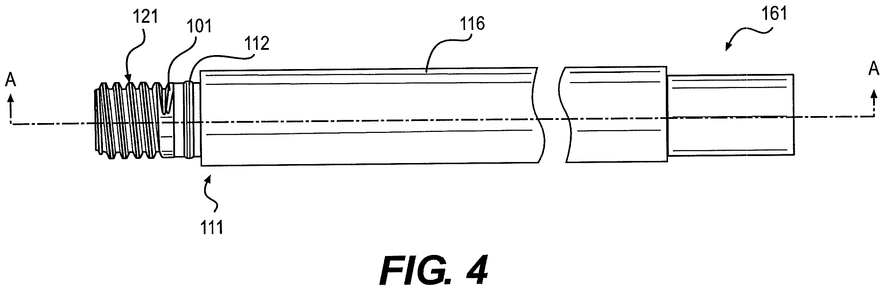

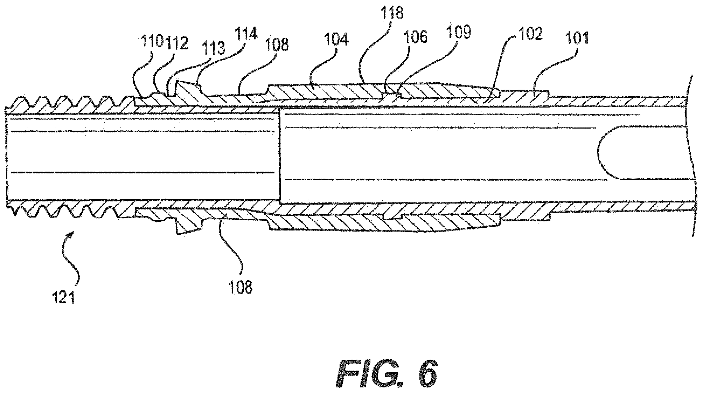

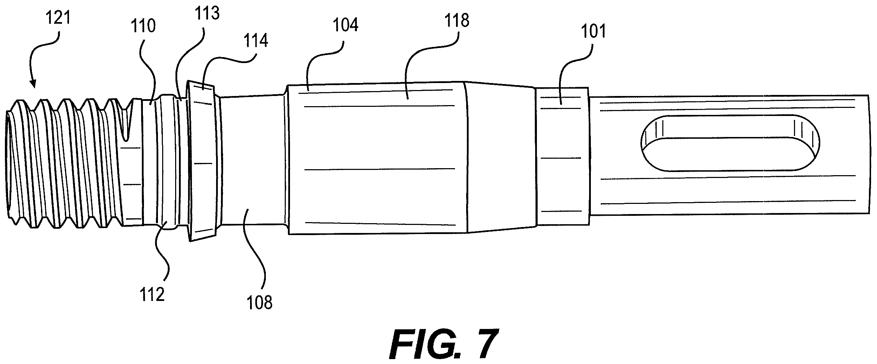

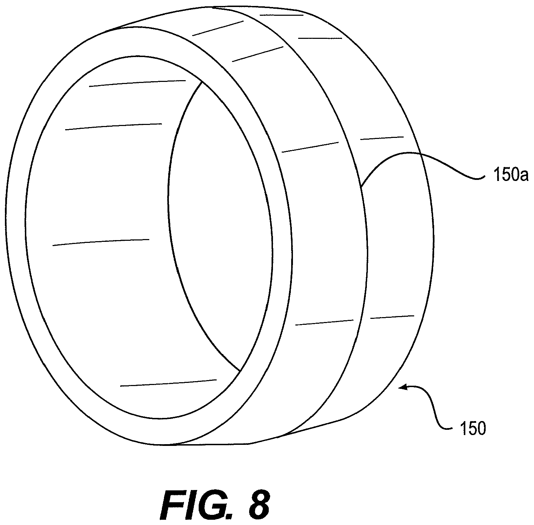

FIGS. 3-8 show an exemplary embodiment of components of a tube end assembly 111 of a reusable handpiece 123 in accordance with a non-limiting aspect of the disclosure. The tube end assembly 111 includes a tube end 101 having an interface 121 for engaging and/or attaching to the back hub 400 of the tip 20. This interface 121 may include exterior threading (which may threadedly engage with complimentary threads on the back hub 400 of the tip 21). The tube end may be made of stainless steel or other suitable material.

The proximal end of the tube end 101 is configured to attach to a distal end of a tube 161 to form a continuous lumen therewith, through which an actuable shaft may be inserted. The tube end 101 also includes a channel 102 located between the interface 121 and proximal end of the tube end. This channel 102 is configured to receive an extended retention seal 104, which is overmolded to the tube end 101 by injection molding. In this way, the use of epoxy or other adhesives is avoided. The retention seal 104 may be made from polypropylene or any other suitable material, and may have a Rockwell hardness of 93, but materials of other suitable hardnesses may be used. The retention seal 104, tube end 101 and sealing ring 150 (and tube 161) are covered with a fluid-resistant outer sheath 116 which may be heat-shrunk and/or extruded thereover, thereby forming the tube end assembly 111. The outer sheath 116 may be made of polyetheretherketone (PEEK) or other suitable material.

The retention seal 104, once affixed to the tube end 101, is generally flush therewith. The retention seal 104 includes an internal recess 106 for engaging a protrusion 109 on the tube end. In this way, the retention seal 104 is further prevented from movement in the axial direction relative to the tube end 101. The retention seal 104 includes, in this order from the distal direction, a featureless distalmost part 110, a ridge 112, a featureless intermediate part 113, a flange 114, a channel 108 and a proximal part 118 which inwardly tapers in the proximal direction. The diameter of the tube end 101 at the distalmost part may be 0.3937 - 0.4013 cm (0.155 - 0.158 inches), although other suitable dimensions may be used, and the diameter of the tube end at the ridge 112 may be 0.4166 - 0.4216 cm (0.164 - .0166 inches), although other suitable dimensions may be used.

The ridge 112 may have a trapezoidal or rounded cross-section, or may have a cross-section that is a combination of both, e.g., by having rounded sides and a flat uppermost part, the latter of which may have a width in longitudinal direction X of 0.0127 - 0.0178 cm (0.005 - 0.007 inches), although other suitable dimensions may be used. The ridge is configured to engage the inner surface of the back hub 400 of the tip 20 to create a seal therewith, which prevents fluid from entering the instrument at the tip-handpiece connection point (*i.e.*, the "seam") thereof. This engagement also preserves the electrical safety of the instrument, since electricity does not flow where it is not wanted.

The flange 114 inwardly tapers toward a proximal end of the tube end 101, and is affixed to a distalmost end of the outer sheath 116. The flange is configured to restrict movement of the outer sheath relative to the tube end 101. The diameter of the tube end 101 at the flange 114 may be 0.4978 cm (0.196 inches), although other suitable dimensions may be used.

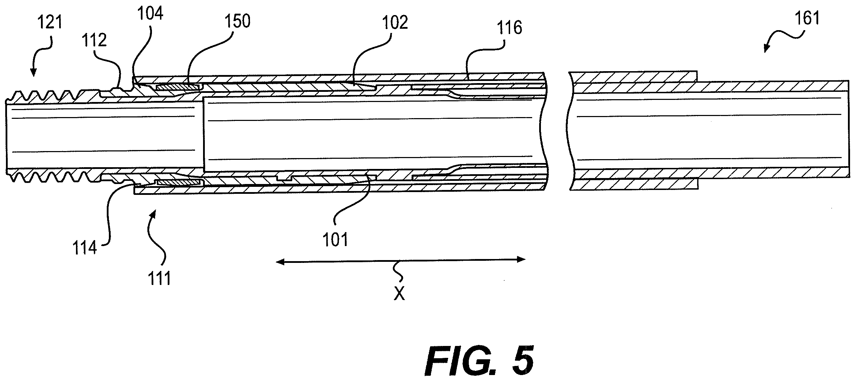

The channel 108 of the retention seal 104 is configured to retain an elastically-deformable sealing ring 150, which is press-fit into the channel, which serves to prevent movement of the seal in the longitudinal direction X relative to the retention seal. Once inserted into the channel 108, the sealing ring 150 is covered (along with the rest of the tube end assembly 111) by the outer sheath 116. Thus, as shown in Fig. 5, the sealing ring 150 is sandwiched between the outer sheath 116 and the retention seal 104, and therefore the sealing ring and at least a portion of the retention seal are sandwiched between the outer sheath 116 and tube end.

The channel 108 may have a width in the longitudinal direction X of 0.2286 cm (0.090 inches), and the sealing ring 150 may have a width in the longitudinal direction X of 0.2032 cm (0.080 inches), although other widths of each of these elements may be used. Once the sealing ring 150 is covered (along with the rest of the tube end assembly 111) by the outer sheath 116, the pressure of the outer sheath against the sealing ring causes the sealing ring to expand in the longitudinal direction X and therefore fill the channel 108, and can form a secure seal therewith without the use of epoxy or other adhesive. Further, rather than forming a right angle in the channel 108, the intersection between the channel walls and channel bottom may be curved.

The sealing ring 150 may have a pentagonal cross section. For example, as shown in Figs. 3, 5 and 8, in the longitudinal direction X the outside of the sealing ring 150 may be downwardly tapered from a highest center point 150a to a lowest point at the edge points of the sealing ring, such that the edge points are flush with the outside of the retention seal 104. The sealing ring 150 may have a Shore durometer of 70, but materials of other suitable durometers may be used. The pentagonal cross section of the sealing ring 150, together with the Shore durometer, allows the sealing ring to be installed in the outer sheath 116 without getting torn or otherwise damaged during the assembly process, and further helps prevent the outer sheath from displacement in the longitudinal direction X relative to the sealing ring, while still form a secure seal between the sealing ring and the outer sheath, without the use of epoxy or other adhesive. Further, the sealing ring 150 may be made out of ethylene propylene diene terpolymer (EPDM); however, other flexible materials may be used. Thus, the retention seal 104 is made of a material which is softer than the tube end 101, and the sealing ring 150 is made of a material which is softer than the retention seal.

A method of assembling the tube end assembly 111 will now be described. First, the extended retention seal 104 is overmolded to the channel 102 of the tube end 101 by injection molding. Then, the sealing ring 150 is press fit into the channel 108 of the retention seal 104. Next, the retention seal 104, tube end 101 and sealing ring 150 (and tube 161) are covered with the outer sheath 116 by heat shrinking and/or extrusion thereover, thereby forming the tube end assembly 111.

In view of the foregoing, the present disclosure, through one or more of its various aspects, embodiments and/or specific features or sub-components, is thus intended to bring out one or more of the advantages as specifically noted below. While the present disclosure includes description with respect to a medical device and procedure, the present invention may be used in a variety of other, non-medical, environments.

The illustrations of the embodiments described herein are intended to provide a general understanding of the structure of the various embodiments, whereby the invention is defined in the appended claims. The illustrations are not intended to serve as a complete description of all of the elements and features of apparatus and systems that
utilize the structures or methods described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

## Claims

1. A handpiece for interfacing with an instrument tip (20), the handpiece (123) comprising:
a tube end (101) comprising an interface at a distalmost end thereof and configured to attach to the instrument tip (20),
**characterized by**
the tube end (101) comprising a retention seal channel (102) about an exterior of the tube end;
a retention seal (104) fixedly positioned in the retention seal channel (102), the retention seal comprising (104) a sealing ring channel (108) about an exterior of the retention seal (104);
an elastically-deformable sealing ring (150) press-fit into the sealing ring channel (108) such that the sealing ring (150) is immovable in a longitudinal direction of the tube end (101); and
an outer sheath (116) positioned about the tube end (101) such that the outer sheath (116) and tube end (101) sandwich the sealing ring (150) and at least a portion of the retention seal (104).

2. The handpiece according to claim 1, wherein the interface (121) is threaded and configured to attach to complimentary threads on the instrument tip (20).

3. The handpiece according to claim 1, wherein the sealing ring (150) has a pentagonal cross section.

4. The handpiece according to claim 1, wherein:
the retention seal (104) is made of a material which is softer than the tube end (101), and
the sealing ring (150) is made of a material which is softer than the retention seal (104).

5. The handpiece according to claim 1, wherein:
the retention seal (104) further comprises a ridge (112) positioned about a circumference of the tube end, the ridge (112) further positioned, in the longitudinal direction of the tube end (101), between a distalmost end of the outer sheath (116) and the interface (121), and
the ridge (112) is configured to engage an inside of a back hub (400) of the instrument tip (20) and create a seal therewith.

6. The handpiece according to claim 1, wherein the retention seal (104) further comprises a flange (114) inwardly tapering toward a proximal end of the tube end (101), the flange (114) positioned at a distalmost end of the outer sheath (116) and configured to restrict movement of the outer sheath (116) relative to the tube end (101).

7. The handpiece according to claim 1, wherein the retention seal (104) is molded to the retention seal channel (102).

8. The handpiece according to claim 1, wherein the retention seal (104) is made of plastic.

9. The handpiece according to claim 1, wherein:
in the longitudinal direction of the tube end (101), the outside of the sealing ring (150) is downwardly tapered from a highest center point, and
a lowest point of the outside of the sealing ring (150) is flush with the outside of the retention seal (104).

10. The handpiece according to claim 1, wherein:
the tube end (101) further comprises a protrusion on the outside thereof,
the retention seal (104) further comprises a recess (106), and
the protrusion engages with the recess.

11. An instrument comprising:
an instrument tip (20); and
a handpiece according to any of claims 1 to 10.

12. The instrument according to claim 11, wherein:
the instrument tip (20) comprises instrument tip threads, and
the interface comprises interface threads such that the interface threads threadedly engage with the instrument tip threads.

13. The instrument according to claim 11, wherein the sealing ring (150) has a pentagonal cross section.

14. The instrument according to claim 11, wherein:
the retention seal (104) is made of a material which is softer than the tube end (101), and
the sealing ring (150) is made of a material which is softer than the retention seal (104).

15. The instrument according to claim 11, wherein:
the instrument tip (20) further comprises a back hub (400),
the retention seal (104) further comprises a ridge (112) positioned about a circumference of the tube end (101), the ridge (112) further positioned, in the longitudinal direction of the tube end (101), between a distalmost end of the outer sheath (116) and the interface, and
the ridge (112) is configured to engage an inside of the back hub (400) and create a seal therewith.

16. The instrument according to claim 11, wherein the retention seal (104) further comprises a flange (114) inwardly tapering toward a proximal end of the tube end (101), the flange (114) positioned at a distalmost end of the outer sheath (116) and configured to restrict movement of the outer sheath (116) relative to the tube end (101).

17. The instrument according to claim 11, wherein the retention seal (104) is molded to the retention seal channel (102).

18. The instrument according to claim 11, wherein the retention seal (104) is made of plastic.

19. The instrument according to claim 11, wherein:
in the longitudinal direction of the tube end (101), the outside of the sealing ring (150) is downwardly tapered from a highest center point, and
a lowest point of the outside of the sealing ring (150) is flush with the outside of the retention seal (104).

20. A method of assembling a handpiece for interfacing with an instrument tip (20) according to any of claims 1 to 10, **characterized by**
affixing the retention seal (104) in the retention seal channel (102) on an outside of a tube end (101);
press-fitting the elastically-deformable sealing ring (150) into a sealing ring channel (108) on an outside of the retention seal (104), such that the sealing ring (150) is immovable in a longitudinal direction of the tube end (101); and
affixing the outer sheath (116) about the tube end such that the outer sheath (116) and tube end (101) sandwich the sealing ring (150) and at least a portion of the retention seal (104).

21. The method of claim 20, wherein the affixing of the retention seal (104) comprises overmolding the retention seal (104) to the outside of the tube end (101).

## Patentansprüche

1. Handstück zur Ankopplung an eine Instrumentenspitze (20), wobei das Handstück (123) umfasst:
ein Rohrende (101), das an einem am weitesten distal gelegenen Ende eine Schnittstelle umfasst und zum Anbringen an der Instrumentenspitze (20) ausgebildet ist,
**dadurch gekennzeichnet, dass**
das Rohrende (101) einen Rückhaltedichtungskanal (102) um eine Außenseite des Rohrendes herum umfasst;
eine Rückhaltedichtung (104), die fest in dem Rückhaltedichtungskanal (102) positioniert ist, wobei die Rückhaltedichtung (104) einen Dichtungsringkanal (108) um eine Außenseite der Rückhaltedichtung (104) herum umfasst;
einen elastisch verformbaren Dichtungsring (150), der in den Dichtungsringkanal (108) derart eingepresst ist, dass der Dichtungsring (150) in Längsrichtung des Rohrendes (101) unbeweglich ist; und
eine äußere Hülle (116), die um das Rohrende (101) herum derart positioniert ist, dass die äußere Hülle (116) und das Rohrende (101) den Dichtungsring (150) und zumindest einen Abschnitt der Rückhaltedichtung (104) zwischen sich einschließen.

2. Handstück nach Anspruch 1, wobei die Schnittstelle (121) mit einem Gewinde versehen und ausgebildet ist, an komplementären Gewindegängen an der Instrumentenspitze (20) angebracht zu werden.

3. Handstück nach Anspruch 1, wobei der Dichtungsring (150) einen fünfeckigen Querschnitt hat.

4. Handstück nach Anspruch 1, wobei:
die Rückhaltedichtung (104) aus einem Material hergestellt ist, das weicher als das Rohrende (101) ist, und
der Dichtungsring (150) aus einem Material hergestellt ist, das weicher als die Rückhaltedichtung (104) ist.

5. Handstück nach Anspruch 1, wobei:
die Rückhaltedichtung (104) ferner eine Wulst (112) umfasst, die um einen Umfang des Rohrendes herum positioniert ist, wobei die Wulst (112) ferner in Längsrichtung des Rohrendes (101) zwischen einem am weitesten distal gelegenen Ende der äußeren Hülle (116) und der Schnittstelle (121) positioniert ist, und
die Wulst (112) ausgebildet ist, um mit einer Innenseite einer hinteren Nabe (400) der Instrumentenspitze (20) in Eingriff zu kommen und mit derselben eine Dichtung zu erzeugen.

6. Handstück nach Anspruch 1, wobei die Rückhaltedichtung (104) ferner einen Flansch (114) umfasst, der sich nach innen in Richtung eines proximalen Endes des Rohrendes (101) verjüngt, wobei der Flansch (114) an einem am weitesten distal gelegenen Ende der äußeren Hülle (116) positioniert und ausgebildet ist, um eine Bewegung der äußeren Hülle (116) relativ zum Rohrende (101) zu beschränken.

7. Handstück nach Anspruch 1, wobei die Rückhaltedichtung (104) auf den Rückhaltedichtungskanal (102) aufgeformt ist.

8. Handstück nach Anspruch 1, wobei die Rückhaltedichtung (104) aus Kunststoff hergestellt ist.

9. Handstück nach Anspruch 1, wobei
in Längsrichtung des Rohrendes (101) die Außenseite des Dichtungsrings (150) von einem höchsten Mittelpunkt ab nach unten verjüngt ist, und ein niedrigster Punkt der Außenseite des Dichtungsrings (150) bündig mit der Außenseite der Rückhaltedichtung (104) ist.

10. Handstück nach Anspruch 1, wobei
das Rohrende (101) ferner einen Vorsprung auf seiner Außenseite umfasst,
die Rückhaltedichtung (104) ferner eine Aussparung (106) umfasst, und der Vorsprung mit der Aussparung in Eingriff kommt.

11. Instrument, umfassend:
eine Instrumentenspitze (20); und
ein Handstück nach einem der Ansprüche 1 bis 10.

12. Instrument nach Anspruch 11, wobei:
die Instrumentenspitze (20) Instrumentenspitzengewindegänge umfasst, und
die Schnittstelle Schnittstellengewindegänge umfasst, derart, dass die Schnittstellengewindegänge in Gewindeeingriff mit den instrumentenspitzengewindegängen kommen.

13. Instrument nach Anspruch 11, wobei der Dichtungsring (150) einen fünfeckigen Querschnitt hat.

14. Instrument nach Anspruch 11, wobei
die Rückhaltedichtung (104) aus einem Material hergestellt ist, das weicher als das Rohrende (101) ist, und
der Dichtungsring (150) aus einem Material hergestellt ist, das weicher als die Rückhaltedichtung (104) ist.

15. Instrument nach Anspruch 11, wobei:
die Instrumentenspitze (20) ferner eine hintere Nabe (400) umfasst,
die Rückhaltedichtung (104) ferner eine Wulst (112) umfasst, die um einen Umfang des Rohrendes (101) herum positioniert ist, wobei die Wulst (112) ferner in Längsrichtung des Rohrendes (101) zwischen einem am weitesten distal gelegenen Ende der äußeren Hülle (116) und der Schnittstelle positioniert ist, und
die Wulst (112) ausgebildet ist, um mit einer Innenseite der hinteren Nabe (400) in Eingriff zu kommen und mit derselben eine Dichtung zu erzeugen.

16. Instrument nach Anspruch 11, wobei die Rückhaltedichtung (104) ferner einen Flansch (114) umfasst, der sich in Richtung eines proximalen Endes des Rohrendes (101) nach innen verjüngt, wobei der Flansch (114) an einem am weitesten distal gelegenen Ende der äußeren Hülle (116) positioniert und ausgebildet ist, um eine Bewegung der äußeren Hülle (116) relativ zu dem Rohrende (101) zu beschränken.

17. Instrument nach Anspruch 11, wobei die Rückhaltedichtung (104) auf den Rückhaltedichtungskanal (102) aufgeformt ist.

18. Instrument nach Anspruch 11, wobei die Rückhaltedichtung (104) aus Kunststoff hergestellt ist.

19. Instrument nach Anspruch 11, wobei:
in Längsrichtung des Rohrendes (101) die Außenseite des Dichtungsrings (150) von einem höchsten Mittelpunkt nach unten verjüngt ist, und ein niedrigster Punkt der Außenseite des Dichtungsrings (150) bündig mit der Außenseite der Rückhaltedichtung (104) ist.

20. Verfahren zum Zusammenfügen eines Handstücks zum Ankoppeln an eine Instrumentenspitze (20) nach einem der Ansprüche 1 bis 10, **gekennzeichnet durch**
Fixieren der Rückhaltedichtung (104) in dem Rückhaltedichtungskanal (102) auf einer Außenseite eines Rohrendes (101);
Einpressen des elastisch verformbaren Dichtungsrings (150) in einen Dichtungsringkanal (108) auf einer Außenseite der Rückhaltedichtung (104), derart, dass der Dichtungsring (150) in Längsrichtung des Rohrendes (101) unbeweglich ist; und
Befestigen der äußeren Hülle (116) um das Rohrende herum, derart, dass die äußere Hülle (116) und das Rohrende (101) den Dichtungsring (150) und mindestens einen Abschnitt der Rückhaltedichtung (104) zwischen sich einschließen.

21. Verfahren nach Anspruch 20, wobei das Befestigen der Rückhaltedichtung (104) das Aufformen der Rückhaltedichtung (104) auf die Außenseite des Rohrendes (101) umfasst.

## Revendications

1. Instrument à main destiné à faire l'interface avec un embout d'instrument (20), ledit instrument à main (123) comprenant :
une extrémité de tube (101) comprenant une interface à une extrémité la plus distale de celui-ci et configurée pour être fixée sur l'embout d'instrument (20),
**caractérisé en ce que**
l'extrémité du tube (101) comprend un canal de joint de rétention (102) sur la périphérie extérieure d'une extrémité du tube (101) ;
un joint de rétention (104) positionné en étant fixé dans le canal de joint de rétention (102), ledit joint de rétention (104) comprenant un canal de bague d'étanchéité (108) sur la périphérie extérieure du joint de rétention (104) ;
une bague d'étanchéité élastiquement déformable (150) emboitée dans le canal de bague d'étanchéité (108), de telle sorte que la bague d'étanchéité (150) n'est pas mobile dans une direction longitudinale de l'extrémité du tube (101) ; et
une gaine extérieure (116) placée autour de l'extrémité du tube (101), de telle sorte que la gaine extérieure (116) et l'extrémité du tube (101) prennent en sandwich la bague d'étanchéité (150) et au moins une partie du joint de rétention (104).

2. Instrument à main selon la revendication 1, dans lequel l'interface (121) est filetée et configurée pour fixer des filetages complémentaires sur l'embout d'instrument (20).

3. Instrument à main selon la revendication 1, dans lequel :
la bague d'étanchéité (150) a une section transversale en forme de pentagone.

4. Instrument à main selon la revendication 1, dans lequel :
le joint de rétention (104) est composé d'un matériau qui est plus mou que l'extrémité du tube (101), et
la bague d'étanchéité (150) est composée d'un matériau qui est plus mou que le joint de rétention (104).

5. Instrument selon la revendication 1, dans lequel :
le joint de rétention (104) comprend en outre une arête (112) placée autour d'une circonférence de l'extrémité du tube (101), l'arête (112) étant en outre placée, dans la direction longitudinale de l'extrémité du tube (101), entre une extrémité la plus distale de la gaine extérieure (116) et l'interface, et
l'arête (112) est configurée pour s'engager à l'intérieur d'un moyeu arrière (400 et créer une étanchéité avec celui-ci.

6. Instrument selon la revendication 1, dans lequel le joint de rétention (104) comprend en outre une bride (114) effilée vers l'intérieur en direction d'une extrémité proximale de l'extrémité du tube (101), la bride (114) étant placée au niveau d'une extrémité la plus distale de la gaine extérieure (116) et configurée pour limiter le déplacement de la gaine extérieure (116) par rapport à l'extrémité du tube (101).

7. Instrument à main selon la revendication 1, dans lequel le joint de rétention (104) est moulé sur le canal de joint de rétention (102).

8. Instrument à main selon la revendication 1, dans lequel le joint de rétention (104) est composé de plastique.

9. Instrument à main selon la revendication 1, dans lequel :
dans la direction longitudinale de l'extrémité du tube (101), l'extérieur de la bague d'étanchéité (150) est effilé vers le bas à partir d'un point central le plus haut, et
un point le plus bas de l'extérieur de la bague d'étanchéité (150) affleure l'extérieur du joint de rétention (104).

10. Instrument à main selon la revendication 1, dans lequel :
l'extrémité du tube (101) comprend en outre une saillie sur sa partie extérieure,
le joint de rétention (104) comprend en outre un retrait (106), et
la saillie s'engage dans le retrait.

11. Instrument comprenant :
un embout d'instrument (20) ; et
un instrument à main selon l'une quelconque des revendications 1 à 10.

12. Instrument selon la revendication 11, dans lequel :
l'embout d'instrument (20) comprend des filetages d'embout d'instrument, et
l'interface comprend des filetages d'interface de telle sorte que les filetages d'interface s'engagent par vissage sur les filetages de l'embout d'instrument.

13. Instrument selon la revendication 11, dans lequel la bague d'étanchéité (150) a une section transversale en forme de pentagone.

14. Instrument selon la revendication 11, dans lequel :
le joint de rétention (104) est composé d'un matériau qui est plus mou que l'extrémité du tube (101), et
la bague d'étanchéité (150) est composée d'un matériau qui est plus mou que le joint de rétention (104).

15. Instrument selon la revendication 11, dans lequel :
l'embout de l'instrument (20) comprend en outre un moyeu arrière (400),
le joint de rétention (104) comprend en outre une arête (112) placée autour d'une circonférence de l'extrémité du tube (101), l'arête (112) étant placée, dans la direction longitudinale de l'extrémité du tube (101), entre une extrémité la plus distale de la gaine extérieure (116) et l'interface, et
l'arête (112) est configurée pour s'engager à l'intérieur du moyeu arrière (400 et créer une étanchéité avec celui-ci.

16. Instrument selon la revendication 11, dans lequel le joint de rétention (104) comprend en outre une bride (114) effilée vers l'intérieur en direction d'une extrémité proximale de l'extrémité du tube (101), la bride (114) étant placée au niveau d'une extrémité la plus distale de la gaine extérieure (116) et configurée pour limiter le déplacement de la gaine extérieure (116) par rapport à l'extrémité du tube (101).

17. Instrument selon la revendication selon la revendication 11, dans lequel le joint de rétention (104) est moulé sur le canal de joint de rétention (102).

18. Instrument selon la revendication 11, dans lequel le joint de rétention (104) est composé de plastique.

19. Instrument selon la revendication 11, dans lequel :
dans la direction longitudinale de l'extrémité du tube (101), l'extérieur de la bague d'étanchéité (150) est effilé vers le bas, à partir du point central le plus haut, et
un point le plus bas de l'extérieur de la bague d'étanchéité (150) affleure l'extérieur du joint de rétention (104).

20. Procédé d'assemblage d'un instrument à main destiné à faire l'interface avec un embout d'instrument (20) selon l'une quelconque des revendications 1 à 10, **caractérisé par**
la fixation du joint de rétention (104) dans le canal de joint de rétention (102) sur l'extérieur d'une extrémité du tube (101) ;
l'emboîtement de la bague d'étanchéité élastiquement déformable (150) dans un canal de bague d'étanchéité (108) sur l'extérieur du joint de rétention (104), de telle sorte que la bague d'étanchéité (150) n'est pas mobile dans une direction longitudinale de l'extrémité du tube (101) ; et
la fixation de la gaine extérieure (116) autour de l'extrémité du tube, de telle sorte que la gaine extérieure (116) et l'extrémité du tube (101) prennent en sandwich la bague d'étanchéité (150) et au moins une partie du joint de rétention (104).

21. Procédé selon la revendication 20, dans lequel la fixation du joint de rétention (104) comprend le surmoulage du joint de rétention (104) sur l'extérieur de l'extrémité du tube (101).
